# EUROPEAN PATENT APPLICATION

(11) **EP 1 867 315 A1**
(43) Date of publication of application: **19.12.2007**
(21) Application number: 07011690.0
(22) Date of filing: 14.06.2007
(51) Int. Cl.: A61H 33/00, C02F 1/48, A61H 9/00

(54) **Device for treating water with a magnetic field**

(30) Priority: 16.06.2006 ES 200601716
(71) Applicant: Universidad Cardenal Herrera-Ceu, Valencia (ES); Deval S.I, S.L, 46007 Valencia (ES); Banos 10, S.L., Castellon (ES); Mollar Capdevila, Juan Jose, Castellon (ES); Rogla Benedito, Vicente, 46001 Valencia (ES); Ventura Remolar, Jose, Castellon (ES); Fortuno Ferriols, Jose Manuel, Castellon (ES)
(72) Inventor: Corpa Arenas, Juan Manuel, 46022 Valencia (ES); Romero Comez, Javier, 46111 Rocafort (ES); Mollar Capdevila, Juan Jose, 12540-Villareal (Castellon) (ES); Rogla Benedito, Vicente, 46001 Valencia (ES); Ventura Remolar, Jose, 12200 Bechi (Castellon) (ES); Fortuno Ferriols, Jose Manuel, 12540 Villareal (Castllon) (ES)
(74) Representative: Soler Lerma, Santiago

(57) **Abstract**

The invention describes a device that can generate a magnetic field, such as a solenoid or magnet, with a shape and insulation allowing it to be crossed by a liquid which will be polarised. The magnetic field generated is regular so that its effect on the liquid passing through the device will be uniform. Due to its preferably tubular shape the device may be fitted in any liquid distribution system or it can even, due to its tubular shape, replace a segment of said circuits.

## Description

The present invention, as indicated by its title, consists of a device for generating circular magnetic fields, such as a magnet or solenoid, with geometry and isolation conditions that allow a liquid or fluid such as water to flow inside it, polarising this liquid.

The applications of these devices are numerous, as they can be used in any type of installation in which it is advantageous to have an outlet of polarised liquid, mainly water, examples of which are bath tubs, shower columns or water massage columns among others.

### BACKGROUND OF THE INVENTION

The use of magnets or solenoids to polarise water or other liquids or fluids is not new, and has been used since Ancient Greece, where it was considered to be curative and healthy.

Its most common form of use is in containers such as bath tubs.

US Patent 5758369 refers to a container or bath tub in which the container itself has inlet orifices that polarise the water and outlet orifices such that the water follows a closed circuit.

Patent PCT/US97/20972 refers to a bath tub in which devices that can generate magnetic fields are disposed on both of its sides but not in contact with the water.

However, currently known patents relating to devices for polarising a liquid such as water describe complex systems and fixed installations that require a complex assembly, which makes them impractical, particularly considering space limitations in homes and the search for accessories that are as versatile as possible.

On the other hand, in the aforementioned patents in order for the effects of the polarised water to act on the user, the latter must be immersed in the water, so that none provide solutions allowing the fulfilment of polarised water, for example, in showers.

In addition to the aforementioned solutions, polarising devices are also known consisting of magnets inserted in the pipes carrying the fluids. However, this technique can only be used with magnets, not with solenoids, with the corresponding limitations, particularly regarding controlling the magnetic field intensity.

### DESCRIPTION OF THE INVENTION

To solve the problems mentioned above, the invention herein described consists essentially of a magnetic field generating device such as a magnet or a solenoid, inside which runs a liquid, preferably water, such that said liquid is polarised.

To make the polarisation regular and controllable, a device is considered that generates a circular field such that the water flow inside it is evenly affected across the whole device.

As conceived, the device must allow a fixed or variable intensity. Thus, for a fixed intensity a device comprising a magnet can be used and for a variable intensity a solenoid can be used.

The type of field created can be continuous, pulsating or alternating according to the desired effect to be obtained by polarising the liquid.

The obvious advantage of the device compared to the aforementioned patents is that this device can be fitted on a pipe or water outlet, form the pipe or water outlet itself or form part of the water distribution circuit such that the water is polarised, so that the beneficial effects of the polarised water can be enjoyed without having to be immersed in the water, simply by installing the polarising device in a shower or water massage column allowing the user to enjoy the beneficial properties of polarised water.

### DESCRIPTION OF A PREFERRED EMBODIMENT OF THE INVENTION

The invention as described will include the various possible uses, whether in a common pipe, a water massage nozzle, a shower head or a point of the water distribution circuit.

Depending on the type of magnetic field desire, with fixed or variable intensity, continuous or alternating, either a magnet or a solenoid can be used.

The use of solenoids is recommended in case it should be desired to regulate the field intensity, as magnets do not allow varying the intensity.

This device, if it is a solenoid that requires connection to an electrical power grid, is provided with a sufficient insulator to prevent electricity from passing into the liquid stream.

The shape of the device disclosed with the above-stated insulation precautions allows the desired liquid, mainly water, to pass through it.

The magnetic field created by the device is circular, so that it has a very regular incidence on the liquid stream, favouring a uniform polarisation of the liquid.

This type of devices can be fitted in a pipe, such as water pipe, or at an outlet or faucet.

The effect obtained when the device is fitted on the outlet point of the water, for example, is that the positive effects of the polarisation will be transmitted to the user without the user having to be submerged in the liquid, instead a shower can be used to obtain said effects.

Therefore, a preferred embodiment of the invention would be a device with a preferably tubular shape inside which is housed a solenoid, such that a stream of liquid, preferably water, is made to pass through the central gap of the solenoid which essentially coincides with the internal area of the solenoid, such that it is subjected to the magnetic field generated and is polarised.

Depending on the type of action preferred and the polarisation level required, it is possible to vary the intensity of the magnetic field of the solenoid with a simple potentiometer operated by the user.

The final shape and size of the device will be determined by its intended function and the size of the solenoid calculated for the required uses.

This embodiment of the invention is not limiting, as the device can be constituted by a magnet, in which case it will lack a potentiometer and the insulation will not be necessary.

## Claims

1. IMPROVED MAGNETIC FIELD GENERATING DEVICE of the type of those essentially using a solenoid or a magnet to polarise the water, essentially **characterised in that** its shape and insulation conditions allow a liquid or fluid to pass through it so that it is polarised.

2. IMPROVED MAGNETIC FIELD GENERATING DEVICE according to the previous claim, essentially **characterised in that** it generates a circular magnetic field that affects the liquid passing through it in a regular and uniform manner.

3. IMPROVED MAGNETIC FIELD GENERATING DEVICE according to previous claims, essentially **characterised in that** it is fitted in the water outlet or faucet of a shower, bath or water massage system, this faucet optionally being constituted by the device itself.

4. WATER DISTRIBUTION SYSTEM COMPONENT of the type consisting of a tubular body one of the ends of which is connected to a water distribution system and the other being free, ejecting the water, essentially **characterised in that** said tubular body is constituted by a solenoid or a magnet according to claims 1 and 2.

5. WATER DISTRIBUTION SYSTEM COMPONENT of the type with a preferably tubular shape used to join to pipe segments in the distribution circuit of water or another liquid, essentially **characterised in that** said preferably tubular element consists of a solenoid or a magnet according to claims 1 and 2.

6. WATER DISTRIBUTION SYSTEM COMPONENT of the type allowing to mix air and water usually employed in water massage installations, essentially **characterised in that** either in its water inlet or its water outlet a solenoid or magnet is inserted according to claims 1 and 2.
